# EUROPEAN PATENT APPLICATION

(11) **EP 4 450 032 A1**
(43) Date of publication of application: **23.10.2024**
(21) Application number: 24159767.3
(22) Date of filing: 26.02.2024
(51) Int. Cl.: A61F 5/37, A61G 7/10

(54) **KNEE STRAP TO AID RISING POST ABDOMINAL SURGERY**

(30) Priority: 22.04.2023 IE S20230117
(71) Applicant: Rossiter, Hazel, Droghea County Louth A92HEF8 (IE)
(72) Inventor: Rossiter, Hazel, Droghea County Louth A92HEF8 (IE)

(57) **Abstract**

Knee strap for post abdominal surgery, comprising of a small handle, buckle and velcro close

## Description

### Field of the invention

The present invention relates to a knee strap, in particular for helping an individual rise up out of a chair or bed post abdominal surgery.

### Background to the invention

There are currently no straps available that specifically help support individuals post abdominal surgery, to help them rise up out of bed or off an armchair, without feeling severe pain in their abdomen.

Patients who undergo abdominal surgery, such as a caesarean section, can struggle to rise up because this requires use of the abdominal muscles in order to rise.

The less pressure put on abdominal muscles the better for recovery and mental health.

### Statement of invention

The invention consists of a soft strap with a velcro close. The strap has a small soft handle attached on the top and the strap is placed above the knee. The handle on the strap is designed to assist patients in safely transitioning from a seated or lying position to a standing position following abdominal surgery. The strap helps support the patient, allowing patients to sit up or rise from a chair or bed with greater ease and comfort. The patient would grab the handle on the strap to pull their torso up and forward using the strength in their arm rather than putting pressure on the abdomen.

This is a strap that would be of huge benefit to patients post caesarean section and it would mean less reliance on the midwives and nurses to assist patients to sit up or rise from a chair or bed, therefore putting less stress on the HSE.

### Brief description of the drawings

The invention will be more clearly understood from the following description of an embodiment thereof, given by way of an example only, with reference to the accompanying drawings, in which:

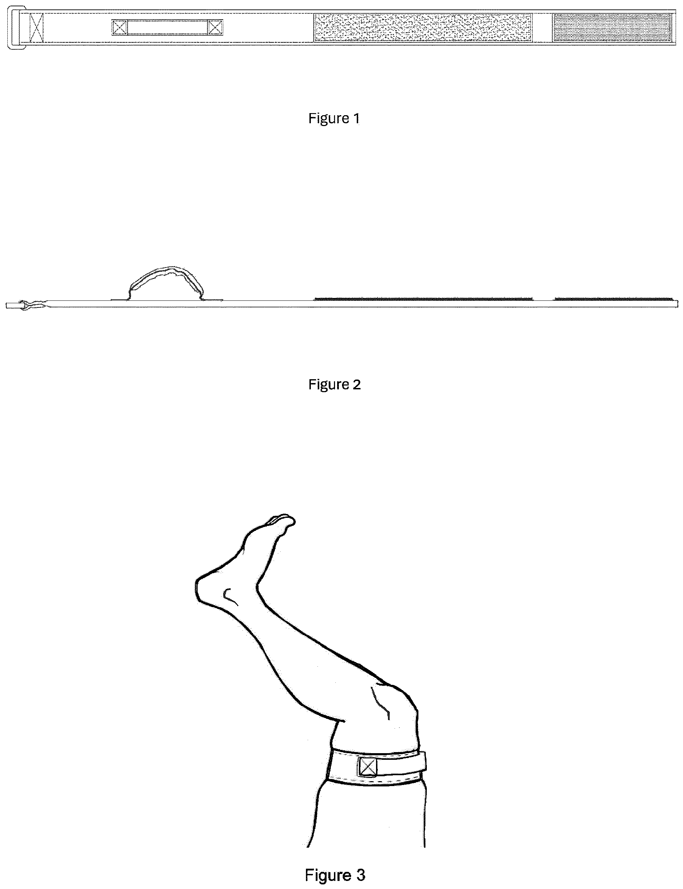

- FIGURE 1 shows a top-down view of the strap
- FIGURE 2 shows a side view of the strap to show the handle attached
- FIGURE 3 shows a side view of the strap on the leg
- FIGURE 4 shows a back of the leg view of the strap to show the handle, buckle and Velcro close over.

### Detailed description

The proposed strap consists of a strap of a length of material with a buckle (2a) positioned at one end and a Velcro closing mechanism (3a) on the other end. The Velcro closing mechanism consists of two strips of Velcro positioned on the outer side of the strap. A handle (1a) is positioned along the length of the strap on the outer side. To close the strap around a user's leg, the Velcro closing mechanism (3a) is looped through the buckle (2a) and doubled back over on itself to press both strips against each other, therefore ensuring the hooks of the Velcro are locked around the loops of the Velcro and the strap is secured in position.

This strap is placed above the knee. The strap loops through a plastic buckle and folds back on top of Velcro to close. The handle (1a) will consist of soft material and be used to pull oneself up by putting all body weight to the strap rather than putting weight to the abdominal muscles.

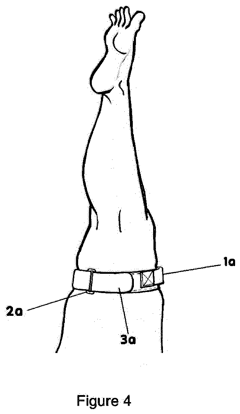

Referring now to FIGURE 4 - The strap encompasses a small soft handle (1a) that is used to lift oneself upwards when sitting down or lying back on your bed.

The strap must be secured tight by putting the end of the strap through the buckle (2a) and securing by doubling the strap over onto the Velcro (3a). This strap can be worn over clothing or directly onto your skin.

Without this strap and when rising from a chair or bed, patients will put pressure on the abdominal muscles causing pain to be felt and making it harder to rise and heal.

## Claims

1. A strap for assisting a patient in transitioning from a seated or lying down position to a standing position comprising:
a strap of a length of material with a buckle (2a) positioned at one end and a Velcro closing mechanism (3a) on the other end, wherein the Velcro closing mechanism consists of two strips of Velcro positioned on the outer side of the strap;
a handle (1a) is positioned along the length of the strap, on the outer side, wherein in use the Velcro closing mechanism (3a) is looped through the buckle (2a) and doubled back over on itself to press both strips against each other, therefore ensuring the hooks of the Velcro are locked around the loops of the Velcro and the strap is secured in position.

2. A method for assisting a patient to transition from a seated or lying down position into an upright position using a strap as claimed in claim 1 comprising the step of:
placing fingers around the handle of the strap and using arm to pull oneself upwards,
therefore avoid using the abdominal muscles when rising.
